(19) Europäisches Patentamt
European Patent Office
Office européen des brevets

(11) Numéro de publication: **0 215 687**
**B1**

(12) # FASCICULE DE BREVET EUROPEEN

(45) Date de publication du fascicule du brevet: **23.05.90**

(51) Int. Cl.⁵: **C07D 233/88,** A61K 35/56

(21) Numéro de dépôt: **86401600.1**

(22) Date de dépôt: **17.07.86**

(54) **Substance biologiquement active, appelée girolline, extraité de l'éponge Pseudaxinyssa cantharella, son procédé de préparation et les compositions pharmaceutiques qui la contiennent.**

(30) Priorité: **18.07.85 FR 8510997**

(43) Date de publication de la demande:
**25.03.87 Bulletin 87/13**

(45) Mention de la délivrance du brevet:
**23.05.90 Bulletin 90/21**

(84) Etats contractants désignés:
**AT BE CH DE FR GB IT LI LU NL SE**

(56) Documents cités:
**Néant**

(73) Titulaire: **RHONE-POULENC SANTE, 20, avenue Raymond Aron, F-92160 Antony(FR)**

(72) Inventeur: **Ahond, Alain, 66 rue Galliéni, F-92240 Malakoff(FR)**
Inventeur: **Laboute, Pierre, Centre ORSTOM BP A5, Noumea Cédex Nouvelle-Calédonie(FR)**
Inventeur: **Laurent, Dominique, Centre ORSTOM BP A5, Noumea Cédex Nouvelle-Calédonie(FR)**
Inventeur: **Potier, Pierre, 14, avenue de Breteuil, F-75007 Paris(FR)**
Inventeur: **Poupat, Christiane, 11 rue des Peupliers, F-78370 Plaisir(FR)**
Inventeur: **Pusset, Jacques, 17 route de Villeconin Saint-Yon, F-91650 Breuillet(FR)**
Inventeur: **Pusset née Thillou, Michèle, 17 route de Villeconin Saint-Yon, F-91650 Breuillet(FR)**
Inventeur: **Thoison, Odile, 6 rue de l'Ormeteau, F-91420 Morangis(FR)**

(74) Mandataire: **Pilard, Jacques et al, RHONE-POULENC RECHERCHES Service Brevets Pharma 25, Quai Paul Doumer, F-92408 Courbevoie Cedex(FR)**

**EP 0 215 687 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne une substance biclogiquement active, son procédé de préparation et les compositions pharmaceutiques qui la contiennent.

La substance, désignée sous le nom de girolline est extraite d'une éponge identifiée à Pseudaxinyssa cantharella dont les caractéristiques ont été décrites par C. Lévi, Bull. Mus. Natn. Hist. Nat. Paris, 4ème Série, 5, 719-722 (1983) et qui peut être pêchée dans le lagon de Nouméa (Nouvelle Calédonie).

La girolline se présente sous la forme d'une poudre blanche très hygroscopique.

La structure de la girolline, sous forme de base ou de sel, peut être présentée par la formule suivante:

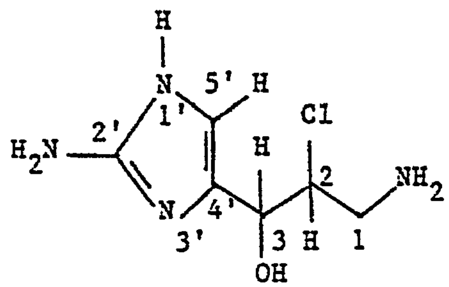

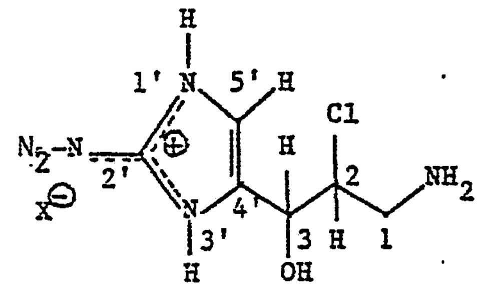

dans laquelle $X^{\ominus}$ représente un anion tel que l'anion chlorure ($Cl^-$).

La structure de la girolline est déterminée à partir de ses spectres de résonance magnétique nucléaire ($^1H$, $^{13}C$, $^{15}N$) et de masse et des spectres de ses dérivés.

La structure de la chaîne hydrocarbonée est déterminée par les spectres de résonance magnétique nucléaire ($^1H$ et $^{13}C$) de la girolline (400 et 100,1 MHz en solution dans $D_2O$ avec, comme référence externe, le dioxanne -TMS pour $^{13}C$ et $D_2O$ à 4,83 ppm pour $^1H$) qui présentent les caractéristiques suivantes:

– C (1) –$H_2$ $\delta c$ = 44,40 ppm (t, J = 147 Hz)
$\delta H$ = 3,48 ppm (dd, J = 13,7 et 9,4 Hz) et
3,64 ppm (dd, J = 13,7 et 3,3 Hz)

– C (2)–H $\delta c$ = 61,2 ppm (dd, J = 154 et 4 Hz)
$\delta_H$ = 4,62 ppm (dt, J = 9,4 et 3,3 Hz)

– C (3)–H $\delta_c$ = 67,3 ppm (d, J = 146 Hz)
$\delta_H$ = 5,19 ppm (dd, J = 3,3 et 1 Hz)

= C (5')–H $\delta c$ = 113,25 ppm (d, J = 202 Hz)
$\delta H$ = 6,80 ppm (d, J = 1 Hz)

= C (4') $\delta c$ = 126,25 ppm (d ép., J = 6 Hz)

= C (2') $\delta c$ = 148,03 ppm (d ép., J = 7 Hz)

Le spectre de résonance magnétique nucléaire de $^{15}N$ (40 MHz en solution dans $D_2O$) donne trois signaux à 47,6 ; 71,4 et 151,3 ppm.

Le spectre de masse de la girolline permet de confirmer la présence d'un atome de chlore lié au squelette:

D.C.I. ($NH_3$) → 191 et 193: présence d'un Cl(C)
D.C.I. ($ND_3$) → 198: présence de 6H interchangeables
F.A.B. (Xe) → 191 et dimère à 381.

Les dérivés ayant permis de confirmer la présence d'oxygène ont été préparés de la manière suivante:

1° Dérivés "adamantyles".

Une solution de 15 mg (0,08 m.mole) de girolline dans 5 cm3 d'eau est amenée à pH de 8-9 par addition d'une solution de bicarbonate de sodium à 10 %. On ajoute alors, en 3 fois, 0,24 m.mole de fluoroformiate d'adamantyle en solution dans le dioxanne (3 fois 0,3 cm3). Après 6 heures à une température voisine de 20-23°C, le mélange réactionnel est extrait à l'éther éthylique. Les extraits organiques sont purifiés par

chromatographie sur couche épaisse (silice ; chlorure de méthylène/acétate d'éthyle 1-1 en volumes). On isole de cette manière deux produits:

a) un produit moins polaire (7,3 mg) dont les caractéristiques sont les suivantes:
Rf = 0,81
$[\alpha]_D$ = + 12,6 (C = 3,1 ; chloroforme)
spectre de masse: F.A.B. → 725 et 727 (isotope Cl)
spectre de résonance magnétique nucléaire du proton
(200 MHz, en solution dans $CDCl_3$):

| | |
|---|---|
| adamantyle | $\delta_H$ = 1,70 et 2,25 ppm (2 m) |
| -C (1)-$H_2$ | $\delta_H$ = 3,48 ppm (m) |
| -C (2)-H | $\delta_H$ = 4,47 ppm (m) |
| -C (3)-H | $\delta_H$ = 5,67 ppm (d, J = 6 Hz) |
| =C (5')-H | $\delta_H$ = 6,96 ppm (s) |
| -N-H | $\delta_H$ = 5,30 et 5,87 ppm (m de 1H et 2H) |

L'étude de ce spectre montre que le produit obtenu est trisubstitué.

b) un produit plus polaire (9 mg) dont les caractéristiques sont les suivantes:
Rf = 0,27
spectre de masse: F.A.B. → 547 et 549 (isotope Cl)
spectre de résonance magnétique nucléaire du proton (200 MHz, en solution dans $CDCl_3$):

| | |
|---|---|
| adamantyle | $\delta_H$ = 1,67 et 2,18 ppm (2m) |
| -C(1)-$H_2$ | $\delta_H$ = 3,40 et 3,73 ppm (2m) |
| -C(2)-H | $\delta_H$ = 4,37 ppm (m) |
| -C(3)-H | $\delta H$ = 4,77 ppm (d, J = 2 Hz) |
| = C(5')-H | $\delta_H$ = 6,96 ppm (s) |
| -N-H et -O-H | $\delta_H$ = 4,46 ; 5,50 et 5,90 ppm (3m) |

L'étude de ce spectre montre que le produit obtenu est disubstitué.

Entre les deux dérivés obtenus, il existe une différence $\Delta\delta_H$ = 1,10 ppm pour -C (3)-H, ce qui permet de conclure à la présence d'un radical hydroxyle en -3.

2° Dérivés "acétylés"

Le dérivé "adamantyle" le plus polaire est acétylé de la manière suivante:

A 10 mg du produit le plus polaire (M → 547-549) dans 0,6 cm3 de pyridine anhydre on ajoute 0,55 cm3 d'anhydride acétique. On agite pendant 2 heures à une température voisine de 20°C. Le mélange réactionnel est versé dans de l'eau glacée puis extrait par du chlorure de méthylène.

Après évaporation du solvant, le produit brut obtenu est purifié par chromatographie en couche épaisse (silice ; chlorure de méthylène/acétate d'éthyle: 1-1 en volumes). On isole ainsi deux produits:

a) dérivé di (0,N) acétylé dont les caractéristiques sont les suivantes:
Rf = 0,63
spectre de masse: i.c. 631 et 633 (isotope Cl)
spectre de résonance magnétique nucléaire du proton (200 MHz, en solution dans $CDCl_3$):

| | |
|---|---|
| adamantyle | $\delta_H$ = 1,65 et 2,18 ppm (2m) |
| $-CO-CH_3$ | $\delta_H$ = 2,28 et 2,47 ppm (2s) |
| $-C(1)-H_2$ | $\delta_H$ = 3,5 ppm (m) |
| -C(2)-H | $\delta_H$ = 4,55 ppm (d ép., J = 6Hz) |
| -C(3)-H | $\delta_H$ = 6,0 ppm (d, J = 6Hz) |
| =C(5')-H | $\delta_H$ = 7,10 ppm (s) |
| -N-H | $\delta_H$ = 5,20 et 9,65 ppm (m) |

b) dérivé mono (O) acétylé dont les caractéristiques sont les suivantes:
Rf = 0,60
spectre de masse: i.c. 589 et 591 (isotope Cl)
spectre de résonance magnétique nucléaire du proton (200 MHz, en solution dans $CDCl_3$):

| | |
|---|---|
| adamantyle | $\delta_H$ = 1,60 et 2,17 ppm (2m) |
| $CO-C-H_3$ | $\delta_H$ = 2,25 ppm (s) |
| $-C(1)-H_2$ | $\delta_H$ = 3,43 ppm (m) |
| -C(2)-H | $\delta_H$ = 4,47 ppm (m) |
| -C(3)-H | $\delta_H$ = 5,85 ppm (d, J = 6 Hz) |
| = C(5')-H | $\delta_H$ = 6,92 ppm (s) |
| -N-H | $\delta_H$ = 5,15 et 5,77 ppm. |

Entre le dérivé monoacétylé et le dérivé adamantylé le plus polaire non acétylé, il existe une différence $\delta_H$ = 1,08 ppm pour le -C(3)-H, ce qui permet de confirmer la présence du radical hydroxyle en - 3.

3° Dérivé dinitrophénylé

a) dérivé bis(dinitro-2,4 phénylé):
A 14 mg (0,07 mmole) de girolline, on ajoute 28 mg de bicarbonate de sodium en solution dans 0,5 cm3 d'eau distillée. Après 30 minutes d'agitation à température ambiante, on ajoute une solution de 112 mg de dinitro-2,4 fluoro-1 benzène dans 2 cm3 d'éthanol.

On agite pendant 2 heures à une température voisine de 20°C. Après concentration à sec, le résidu est repris par 20 cm3 d'un mélange chlorure de méthylène/méthanol (8-2 en volumes). Après filtration sur verre fritté et évaporation à sec du filtrat, le résidu (102 mg) est purifié par flash-chromatographie (silice Merck 7734 ; hauteur de la colonne 150 mm ; diamètre: 20 mm ; pression 0,3 bar ; éluant: chlorure de méthylène/méthanol 8-2 en volumes).

On obtient ainsi le dérivé "bis (dinitro-2,4 phényle)" (15 mg) dont les caractéristiques sont les suivantes:
Rf = 0,23
$[\alpha]_D$ = 0 (méthanol)
spectre de masse: F.A.B.→523 et 525 (isotope Cl)
spectre de résonance magnétique nucléaire du proton (400 MHz, en solution dans $CD_4O$):

| | |
|---|---|
| -C(1)-H : | $\delta_H$ = 3,86 ppm (dd, J = 15 et 7 Hz) |
| -C(1)-H : | $\delta_H$ = 4,0 ppm (dd, J = 15 et 5 Hz) |
| -C(2)-H : | $\delta_H$ = 4,55 ppm (m) |
| -C(3)-H : | $\delta_H$ = 4,80 ppm (d, J = 5Hz) |
| =C(5')-H : | $\delta_H$ = 6,83 ppm (s) |

Les protons des noyaux phénylés sont notés "ou"

$=\underset{|}{C}(6''')-H$ $\delta_H$ = 7,23 ppm (d, J = 9 Hz)
$=\underset{|}{C}(6'')-H$ $\delta_H$ = 7,97 ppm (d, J = 9 Hz)
$=\underset{|}{C}(5''')-H$ $\delta_H$ = 8,35 ppm (dd, J = 9 et 3 Hz)
$=\underset{|}{C}(5'')-H$ $\delta_H$ = 8,70 ppm (dd, J = 9 et 2,5 Hz)
$=\underset{|}{C}(3''')-H$ $\delta_H$ = 8,98 ppm (d, J = 3 Hz)
$=\underset{|}{C}(3'')-H$ $\delta_H$ = 9,08 ppm (d, J = 2,5 Hz)

b) dérivé "bis(dinitro-2,4 phénylé)" acétylé
9 mg du produit "diphénylé" sont dissous dans 2 cm3 d'anhydride acétique et 2 cm3 de pyridine anhydre.
On agite pendant 4 jours à une température voisine de 20°C.
Après évaporation à sec, le résidu est chromatographié sur plaque de silice (Merck 60 F254 ; chlorure de méthylène/méthanol 95-5 en volumes). On isole un composé (8 mg) dont les caractéristiques sont les suivantes:
Rf = 0,60
spectre de résonance magnétique nucléaire du proton (200 MHz, en solution dans $CD_4O$)

$CO-C-H_3$ $\delta_H$ = 1,90 et 2,16 ppm (2s)
$-\underset{|}{\overset{|}{C}}(3)-H$ $\delta_H$ = 6,14 ppm (d, J = 7,5 Hz)
$=\underset{|}{C}(5')-H$ $\delta_H$ = 7,56 ppm (s)

Entre les dérivés "bis(dinitro-2,4 phénylés)" acétylé et non acétylé, il existe une différence $\Delta\ \delta_H$ = 1,34 ppm, ce qui confirme la présence d'un radical hydroxyle en -3.

Selon l'invention, la girolline est obtenue à partir d'extraits d'animaux congelés, broyés et lyophilisés solubles dans l'éthanol et le méthanol. Les extraits sont purifiés par chromatographies successives sur des supports appropriés en éluant avec des solvants ou des mélanges de solvants convenables.

La girolline selon l'invention peut se présenter également sous forme de sels d'addition avec les acides minéraux ou organiques tel que l'acide chlorhydrique.

La girolline selon l'invention présente des propriétés antitumorales remarquables.

In vitro, elle s'est montrée active sur les cellules leucémiques P 388 à des concentrations comprises entre 0,001 et 1 µg/cm3.

L'activité in vitro a été confirmée in vivo chez la souris greffée avec des cellules leucémiques P 388 à des doses voisines de 1 mg/kg par voie intra-péritonéale.

La présente invention concerne également les compositions pharmaceutiques qui contiennent la girolline en association avec tout autre produit pharmaceutiquement acceptable, qu'il soit inerte ou physiologiquement actif.

Ces compositions peuvent être présentées sous toute forme appropriée à la voie d'adminstration prévue. La voie parentérale est la voie d'administration préférentielle et notamment la voie intraveineuse.

Les compositions selon l'invention pour administration parentérale peuvent être des solutions stériles aqueuses ou non aqueuses, des suspensions ou des émulsions. Comme solvant ou véhicule, on peut employer le propylèneglycol, les huiles végétales, on peut particulier l'huile d'olive, et les esters organiques injectables, par exemple l'oléate d'éthyle. Ces compositions peuvent également comprendre des adjuvants en particulier des agents mouillants, émulsifiants et dispersants. La stérilisation peut se faire de plusieurs façons, par exemple à l'aide d'un filtre bactériologique, en incorporant à la composition des agents stérilisants, par irradiation ou par chauffage. Elles peuvent également être préparées sous forme de compositions solides stériles qui peuvent être dissoutes ou dispersées au moment de l'emploi dans de l'eau stérile ou tout autre milieu stérile injectable.

La girolline est particulièrement utile dans le traitement des hémopathies malignes et des tumeurs solides à des doses journalières généralement comprises entre 0,1 et 1 mg/kg par voie intraveineuse pour un adulte.

Les exemples suivants, donnés à titre non limitatif, montrent comment l'invention peut être mise en pratique.

EXEMPLE 1

25 kg d'éponges Pseudaxinyssa cantharella congelées sont broyés et lyophilisés. On obtient ainsi 5 kg d'extrait brut contenant 45 % de chlorure de sodium qui est extrait par 3 fois 6,5 litres d'éthanol. La

phase éthanolique est concentrée à sec. On obtient ainsi 1 kg d'un produit qui est repris par 6 litres de méthanol. La fraction soluble dans le méthanol, après évaporation du solvant, est reprise par 10 litres d'eau. Le résidu sec, issu de la phase aqueuse est repris par 4 litres de méthanol. Après distillation du solvant, on obtient 477 g d'un extrait pâteux qui est adsorbé sur 439 g de silice.

On chromatographie 365 g de l'extrait adsorbé sur silice sur une colonne de 8 cm de diamètre contenant 800 g de gel de silice (60 Merck Type 7734) en éluant avec un mélange initial acétate d'éthyle-méthyléthylcétone-acide formique-eau (5-3-0,5-0,5 en volumes) dans lequel on fait croître les proportions d'acide formique et d'eau et en recueillant des fractions de 400 cm3. Chaque fraction est contrôlée par chromatographie en couche mince, les plaques étant révélées par pulvérisation d'eau de Javel puis, après séchage, par pulvérisation d'une solution saturée d'o-tolidine dans l'eau acidifiée par 2 % d'acide acétique. On recueille 70 fractions qui sont regroupées en 16 lots. Les huit premiers lots qui correspondent aux fractions 1 à 36 ne contiennent que des produits moins polaires que la girolline. Le dixième lot (25,1 g) correspondant aux 5 fractions éluées par le mélange acétate d'éthyle-méthyléthylcétone-acide formique-eau (5-5-2-2 en volumes) est le plus riche en girolline.

On filtre la girolline brute ainsi obtenue sur une colonne de 5 cm de diamètre contenant 800 g de gel de Sephadex LH 20 en éluant avec du méthanol. On obtient ainsi 5,05 g de girolline pure, avec un rendement de 0,5 g par kg d'organisme congelé.

EXEMPLE 2

On prépare une solution contenant 16 mg/cm3 de girolline en dissolvant 1,6 g de ce produit dans du soluté physiologique apyrogène en quantité suffisante pour obtenir 100 cm3. La solution obtenue est répartie aseptiquement en ampoules à raison de 5 cm3 par ampoule. Les ampoules sont scellées ; elles contiennent chacune 80 mg de girolline.

**Revendications pour les états contractants: BE, CH, DE, FR, GB, IT, LI, LU, NL, SE**

1 - Substance biologiquement active, caractérisée en ce qu'elle répond à la formule:

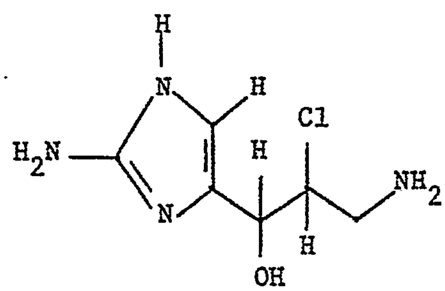

ainsi que ses sels d'addition avec les acides.

2 - Procédé de préparation de la substance selon la revendication 1 caractérisé en ce que l'on purifie par chromatographies successives sur des supports appropriés des extraits solubles dans l'éthanol et le méthanol d'éponge Pseudaxinyssa cantharella congelée, broyée et lyophilisée, et isole le produit obtenue éventuellement sous forme d'un sel d'addition avec un acide.

3 - Composition pharmaceutique caractérisée en ce qu'elle contient une quantité suffisante de la substance selon la revendication 1 en association avec un ou plusieurs produits pharmaceutiquement acceptables inertes ou physiologiquement actifs.

**Revendications pour l'état contractant: AT**

Procédé de préparation d'une substance biologiquement active répondant à la formule:

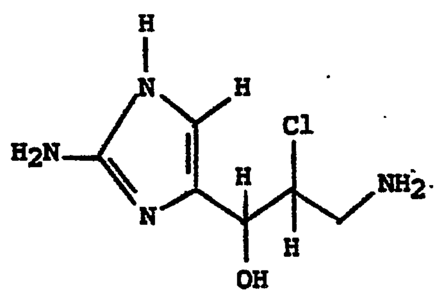

caractérisé en ce que l'on purifie par chromatographies successives sur des supports appropriés des extraits solubles dans l'éthanol et le méthanol d'éponge Pseudaxinyssa cantharella congelée, broyée et lyophilisée, et isole le produit obtenu éventuellement sous forme d'un sel d'addition avec un acide.

**Patentansprüche für die Vertragsstaaten: BE, CH, DE, FR, GB, IT, LI, LU, NL, SE**

1. Biologisch aktive Substanz, dadurch gekennzeichnet, daß sie der Formel

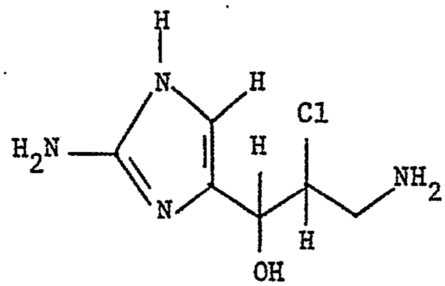

entspricht, sowie ihre Additionssalze mit Säuren.

2. Verfahren zur Herstellung der Substanz gemäß Anspruch 1, dadurch gekennzeichnet, daß man durch aufeinanderfolgende Chromatographien auf geeigneten Trägern in Äthanol und Methanol lösliche Extrakte von gefrorenem, zerkleinertem und lyophilisiertem Schwamm Pseudaxinyssa cantharella reinigt und das erhaltene Produkt gegebenenfalls in Form eines Additionssalzes mit einer Säure isoliert.

3. Pharmazeutische Zusammensetzung, dadurch gekennzeichnet, daß sie eine ausreichende Menge der Substanz gemäß Anspruch 1 zusammen mit einem oder mehreren inerten pharmazeutisch annehmbaren oder physiologisch aktiven Produkten enthält.

**Patentansprüche für den Vertragsstaat: AT**

Verfahren zur Herstellung einer biologisch aktiven Substanz der Formel

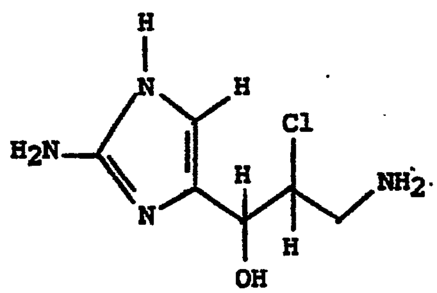

dadurch gekennzeichnet, daß man in Äthanol und Methanol lösliche Extrakte des tiefgekühlten, vermahlenen und gefriergetrockneten Schwammes Pseudaxinyssa cantharella durch aufeinanderfolgende Chromatographien auf geeigneten Trägern reinigt und das erhaltene Produkt gegebenenfalls in Form eines Säureadditionssalzes isoliert.

**Claims for the contracting states: BE, CH, DE, FR, GB, IT, LI, LU, NL, SE**

1. Biologically active substance, characterized in that it corresponds to the formula:

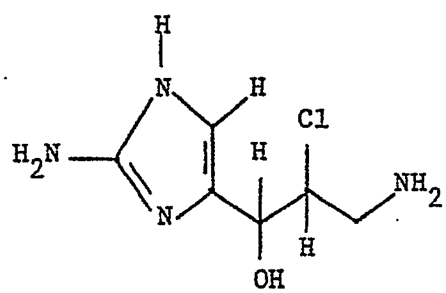

and its addition salts with acids.

2. Process for the preparation of the substance according to claim 1, characterized in that the ethanol- and methanol-soluble extracts of ground and freeze-dried frozen Pseudaxinyssa cantharella sponge are purified by successive chromatography on suitable media, and the product obtained is isolated, if appropriate, in the form of an addition salt with an acid.

3. Pharmaceutical composition characterized in that it contains a sufficient quantity of the substance according to claim 1, in combination with one or more pharmaceutically acceptable products, which are inert or physiologically active.

**Claims for the contracting state: AT**

Process for the preparation of a biologically active substance, corresponding to the formula:

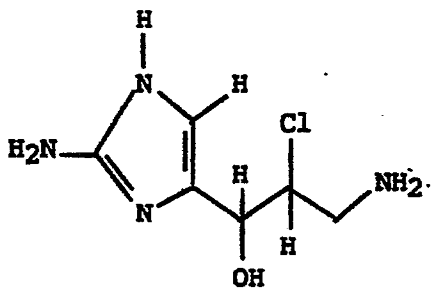

characterized in that the ethanol- and methanol-soluble extracts of ground and freeze-dried frozen Pseudaxinyssa cantharella sponge are purified by successive chromatography on suitable media, and the product obtained is isolated, if appropriate, in the form of an addition salt with an acid.